# EUROPEAN PATENT APPLICATION

(11) **EP 4 814 463 A1**
(43) Date of publication of application: **30.09.2026**
(21) Application number: 24893901.9
(22) Date of filing: 15.10.2024
(51) Int. Cl.: G01L 11/02, G01N 21/59, G01N 21/3504

(54) **GAS MEASUREMENT DEVICE AND GAS MEASUREMENT METHOD**

(30) Priority: 21.11.2023 JP 2023197208
(71) Applicant: Yokogawa Electric Corporation, Musashino-shi, Tokyo 180-8750 (JP)
(72) Inventor: KAWANO Makoto, Musashino-shi, Tokyo 180-8750 (JP); KIMURA Hiroyuki, Shizuoka-shi, Shizuoka 422-8529 (JP)
(74) Representative: Stöckeler, Ferdinand
(86) International application number: PCT/JP2024/036681
(87) International publication number: WO 2025/109907

(57) **Abstract**

A gas measurement device (5) includes an absorbance measurement device (10) configured to irradiate detection light into a reaction vessel (2) that generates methane gas and measure absorbance of the detection light that has passed through a gas atmosphere inside the reaction vessel (2), an absorbance data analysis unit (21) configured to spectrally analyze data of the absorbance measured by the absorbance measurement device (10), a peak detection unit (22) configured to detect a plurality of peak values from the absorbance spectrum analyzed by the absorbance data analysis unit (21), and a pressure calculation unit (23) configured to calculate the pressure inside the reaction vessel (2) based on an intensity ratio of the two peak values in the plurality of peak values detected by the peak detection unit (22).

## Description

### TECHNICAL FIELD

The present invention relates to a gas measurement device and a gas measurement method.

### BACKGROUND ART

The following Patent Document 1 discloses a gas concentration measurement device including a laser configured to oscillate a laser beam with a wavelength and intensity in response to the drive current and temperature, a wavelength stabilizer configured to modulate a drive power supply of the laser with an arbitrary amplitude about a predetermined current value at a predetermined temperature and stabilize the laser beam about an absorption line of a specific gas, a cell for a measurement gas configured to contain a specific gas that is a measurement target and maintains a constant temperature of the specific gas, a photodetector configured to detect an intensity of transmitted light obtained by passing a laser beam of arbitrary amplitude through the cell for a measurement gas, and a measurement means configured to detect a specific component in a signal from the detector using phase-sensitive detection and measure a concentration of the specific gas in the cell for measurement gas based on the change in the detected signal corresponding to the modulated amplitude.

### Citation List

### Patent Document

Patent Document 1: Japanese Unexamined Patent Application, First Publication No. H05-256769

### SUMMARY OF INVENTION

### Technical Problem

Incidentally, for gases like methane, if we focus on one of the absorption spectral lines, since the absorption coefficient has a value that depends on the total atmospheric pressure, when performing concentration measurements in places with drastic barometric pressure changes, such as coal mines and plants, it was necessary to install a separate pressure sensor to observe the pressure and perform corrections based on that value in order to perform accurate concentration measurements.

In the related art described above, instead of not installing the pressure sensor, the above-mentioned problem is solved by installing the cell for a measurement gas that contains the specific gas that is a measurement target and maintains a constant temperature of that specific gas, but, there is a problem in that installing the cell for a measurement gas is costly.

In consideration of the above-mentioned problems, the present invention is directed to providing a gas measurement device and a gas measurement method capable of measuring a specific gas without installing a pressure sensor in a reaction vessel in which a pressure changes.

### Solution to Problem

A gas measurement device according to an aspect of the present invention includes an absorbance measurement device configured to radiate detection light into a reaction vessel that generates a gas and measure an absorbance of the detection light that has passed through a gas atmosphere inside the reaction vessel; an absorbance data analysis unit configured to spectrally analyze data of the absorbance measured by the absorbance measurement device; a peak detection unit configured to detect a plurality of peak values from an absorbance spectrum analyzed by the absorbance data analysis unit; and a pressure calculation unit configured to calculate the pressure inside the reaction vessel based on an intensity ratio of the two peak values of the plurality of peak values detected by the peak detection unit.

In addition, the gas measurement device according to the aspect of the present invention may include a gas concentration calculation unit configured to calculate a concentration of the gas based on the peak value of the absorbance with a specific wavelength analyzed by the absorbance data analysis unit and the pressure inside the reaction vessel calculated by the pressure calculation unit, and a gas concentration output unit configured to output the calculated concentration of the gas which the gas concentration calculation unit calculates.

In the gas measurement device according to the aspect of the present invention, the gas concentration calculation unit may include a gas concentration output unit configured to output the calculated concentration of the gas.

In addition, the gas measurement device according to the aspect of the present invention may include a data checking unit configured to compare table data, which shows the correspondence between the peak value of the absorbance at a predetermined standard pressure stored in advance and the gas concentration, with the peak value of the absorbance.

In addition, in the gas measurement device according to the aspect of the present invention, the gas concentration calculation unit may include a peak value standardization unit configured to convert the peak value of the absorbance at a specific wavelength analyzed by the absorbance data analysis unit into a peak value at a predetermined standard pressure from the pressure inside the reaction vessel calculated by the pressure calculation unit, and normalize the peak value, and the data checking unit may compare the peak value of the standardized absorbance with the table data, which corresponds to the gas concentration and the peak value of the absorbance at the standard pressure stored in advance.

In addition, in the gas measurement device according to the aspect of the present invention, the peak detection unit may detect the peak values of two adjacent points from the absorbance spectrum analyzed by the absorbance data analysis unit.

In addition, in the gas measurement device according to the aspect of the present invention, when the peak detection unit detects multiple pairs of two adjacent peak values within a predetermined wavelength range, the peak detection unit may select the pair with the largest difference between the peak values of two adjacent points.

A gas measurement method according to an aspect of the present invention includes an absorbance measurement process of radiating detection light into a reaction vessel that generates a gas and measuring an absorbance of the detection light that has passed through a gas atmosphere inside the reaction vessel; an absorbance data analysis process of spectrally analyzing data of the absorbance measured in the absorbance measurement process; a peak detection process of detecting a plurality of peak values from the absorbance spectrum analyzed in the absorbance data analysis process; and a pressure calculation process of calculating the pressure inside the reaction vessel based on an intensity ratio of the peak values of two points in the plurality of peak values detected in the peak detection process.

### Advantageous Effects of Invention

According to the aspect of the present invention, it is possible to measure a specific gas without installing a pressure sensor in a reaction vessel in which a pressure changes.

### BRIEF DESCRIPTION OF DRAWINGS

[FIG. 1] A configuration view of a methane generating device according to an embodiment.
[FIG. 2] A graph showing an example of an absorption spectrum of methane gas according to the embodiment.
[FIG. 3] A graph showing a relationship between an intensity ratio (11/12) of two peak values and a pressure according to the embodiment.
[FIG. 4] A graph showing a relationship between methane concentration and absorbance of each pressure according to the embodiment.

### DESCRIPTION OF EMBODIMENTS

Hereinafter, a gas measurement device and a gas measurement method according to an embodiment of the present invention will be described in detail with reference to the accompanying drawings. Hereinafter, first, a summary of the embodiment of the present invention will be described first, followed by detailed description of the embodiment of the present invention.

### [Summary]

In the gas concentration measurement device of Patent Document 1, a laser beam emitted from a semiconductor laser is split by a beam splitter and transmitted to a cell for a standard gas and a cell for a measurement gas. Light transmitted through the cell for a standard gas is received by a detector, and the received signal reaches a bipolar constant current power supply via a lock-in amplifier and an integrator. Then, the bipolar constant current power supply supplies a positive or negative current to a Peltier element to control the temperature of the semiconductor laser and stabilize the emission of the laser beam. Light transmitted through the cell for a measurement gas is received by a photodetector, and the received signal is transmitted to a lock amplifier and a lock-in amplifier. Next, the pressure is determined by signal processing in a divider and a signal processing unit, and then the gas concentration is obtained.

In this way, in the gas concentration measurement device in the related art, it was necessary to install the cell for a measurement gas. Further, in the gas concentration measurement device, to control the output of the semiconductor laser, it was necessary to install separate gas cells for a standard gas and a gas cell for enclosing the standard gas.

The embodiment of the present invention is a gas measurement device and gas measurement method, in which detection light is radiated into a reaction vessel that generates gas, and absorbance of the detection light that has passed through the gas atmosphere inside the reaction vessel is measured. Next, data of the measured absorbance is spectrally analyzed, and two peak values are detected from the spectrum of the analyzed absorbance. Then, the pressure inside the reaction vessel is calculated based on an intensity ratio of the detected two peak values. Accordingly, since the pressure can be estimated from the peak values of the absorbance, there is no need to install the pressure sensor. In addition, there is no need to remove the pressure sensor during heat treatment, such as for sterilization of the reaction vessel. In addition, since there is no need to remove the gas as in gas chromatography, contamination of the reaction vessel with impurities can be prevented. Further, it is possible to accurately estimate a specific gas concentration inside the reaction vessel in which the pressure is changing.

### [Embodiment]

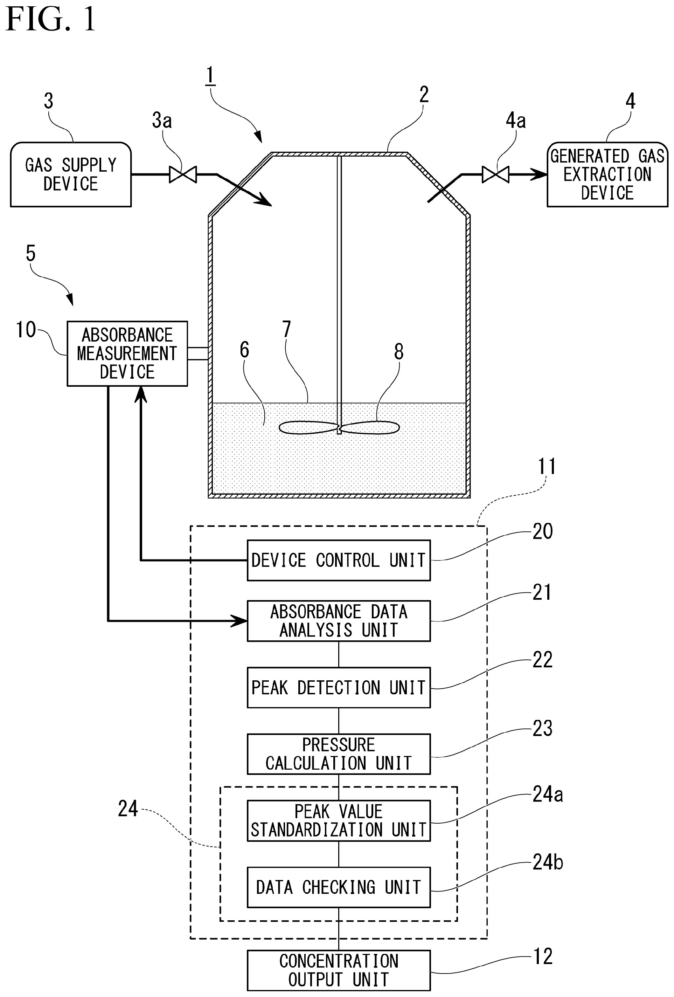

FIG. 1 is a configuration view of a methane generating device 1 according to the embodiment.

As shown in FIG. 1, the methane generating device 1 includes a reaction vessel 2, a gas supply device 3, a generated gas extraction device 4, and a gas measurement device 5.

The reaction vessel 2 cultivates methane-producing bacteria in culture solution 6. Examples of the methane-producing bacteria may include methanobacterium alcaliphilum, methanobacterium bryantii, methanobacterium congolense, methanobacterium defluvii, methanobacterium espanolae, methanobacterium formicicum, methanobacterium ivanovii, methanobacterium palustre, methanobacterium thermaggregans, methanobacterium uliginosum, methanobrevibacter acididurans, methanobrevibacter arboriphilicus, methanobrevibacter gottschalkii, methanobrevibacter olleyae, methanobrevibacter ruminantium, methanobrevibacter smithii, methanobrevibacter woesei, methanobrevibacter wolinii, methanothermobacter marburgensis, methanothermobacter thermoautotrophicus, methanobacterium thermoautotrophicus, methanothermobacter thermoflexus, methanothermobacter thermophilics, methanothermobacter wolfeii, methanothermus sociabilis, methanocorpusculum bavaricum, methanocorpusculum parvum, methanoculleus chikuoensis, methanoculleus submarinus, methanogenium frigidum, methanogenium liminatans, methanogenium marinum, methanomicrobium mobile, methanocaldococcus jannaschii, methanococcus aeolicus, methanococcus maripaludis, methanococcus vannielii, methanococcus voltaei, methanothermococcus thermolithotrophicus, and the like.

In the embodiment, the reaction vessel 2 cultivates strains of methane-producing bacteria such as methanobacteriales, methanomicrobiales, methanocellales, and methanomassillicoccales. These hydrogen-assimilating methane-producing bacteria can also be found in anaerobic groundwater, and methane can be obtained by adding a mixed gas of carbon dioxide (CO₂) and hydrogen gas (H₂) to the anaerobic groundwater containing these bacteria. That is, the reaction vessel 2 contains the culture solution 6 (for example, anaerobic groundwater) which is a culture medium for cultivating methane-producing bacteria. The reaction vessel 2 is under anaerobic conditions, with an oxygen abundance reduced as much as possible.

The gas supply device 3 opens a valve 3a and supplies carbon dioxide and hydrogen gas, which are required by the methane-producing bacteria, to the reaction vessel 2. The gas supply device introduces an H₂/CO₂ mixed gas (for example, 80 vol.%:20 vol.% in a volume ratio) into a gas phase portion inside the reaction vessel 2, pressurizes it to a predetermined pressure, and initiates the cultivation of the methane-producing bacteria.

The generated gas extraction device 4 opens a valve 4a based on the measurement result of the gas measurement device 5, and extracts methane produced by methane-producing bacteria (for example, hydrogen-assimilating methane-producing bacteria). The generated gas extraction device 4 extracts the generated gas from the gas phase portion inside the reaction vessel 2 and stores it in an external tank (not shown). Further, after methane extraction is complete, the valve 4a is closed, and the H₂/CO₂ mixed gas is supplied again from the gas supply device 3 to the reaction vessel 2 to cultivate methane-producing bacteria and generate methane.

It is advisable to supply inert gas into the reaction vessel 2 from the gas supply device 3 or the like before initiating the cultivation of methane-producing bacteria (before supplying the H₂/CO₂ mixed gas to the reaction vessel 2). The reaction vessel 2 can be brought into an anaerobic condition by supplying an inert gas (purging a gas). Examples of the inert gas include nitrogen gas, argon gas, and helium gas.

The reaction vessel 2 is provided with a stirring mechanism 8 configured to stir and mix an area of an air-liquid interface 7 between the culture solution 6 and the H₂/CO₂ mixed gas (reaction gas). The stirring mechanism 8, equipped with a stirring blade, agitates the air-liquid interface 7 of the culture solution 6. Accordingly, dissolution of the reaction gas into the culture solution 6 is promoted. Further, if the area of the air-liquid interface 7 can be increased, for example, a mechanism that vibrates the reaction vessel 2 itself would also suffice.

The gas measurement device 5 measures and outputs the concentration of methane gas generated inside the reaction vessel 2. The measurement result of the gas measurement device 5 is managed by the peripheral device (not shown) of the reaction vessel 2, enabling a long-term operation (long-term generation of methane) of the methane generating device 1. The gas measurement device 5 includes an absorbance measurement device 10, an information processing unit 11, and a concentration output unit 12.

The absorbance measurement device 10 irradiates the reaction vessel 2, which generates methane gas, with detection light and measures the absorbance of the detection light that has passed through the gas phase portion containing gas, i.e., the gas atmosphere inside the reaction vessel 2 (absorbance measurement process). In the embodiment, the measurement target is methane gas, and it is preferable to use at least one of infrared ray and near-infrared ray as the detection light. Further, the measurement target is not limited to methane gas. When inspecting for a specific gas other than methane gas, it is advisable to use a detection light with a wavelength suitable for the specific gas.

The absorbance measurement device 10 can, for example, employ a probe-type device inserted into the reaction vessel 2. The probe-type absorbance measurement device 10 is equipped with a detection light lighting unit and a light receiving unit on the proximal end side of the probe, and a reflecting unit at the tip portion of the probe that reflects the detection light towards the proximal end portion. A ventilation hole is provided between the proximal end portion and the tip portion of the probe, and detection light can be irradiated onto the gas inside the reaction vessel 2 passing through the ventilation hole, and its absorbance can be measured.

The information processing unit 11 includes a device control unit 20, an absorbance data analysis unit 21, a peak detection unit 22, a pressure calculation unit 23, and a gas concentration calculation unit 24 (a peak value standardization unit 24a and a data checking unit 24b). These are separated functionally, but as physical components (hardware), they may be constituted by the same device (for example, an arithmetic processing unit such as a PC) or of multiple devices.

The absorbance data analysis unit 21, the peak detection unit 22, the pressure calculation unit 23, and the gas concentration calculation unit 24 process the measurement data from the absorbance measurement device 10. The device control unit 20 controls the operation of the absorbance measurement device 10 and, based on the processing result of the measurement data from the absorbance measurement device 10, controls the operation of the valve 3a of the gas supply device 3, the valve 4a of the generated gas extraction device 4, and other peripheral devices of the reaction vessel 2 (not shown) mentioned above. Further, while the reaction is in progress, it is advisable to close the valves 3a and 4a to keep the inside of the reaction vessel 2 sealed.

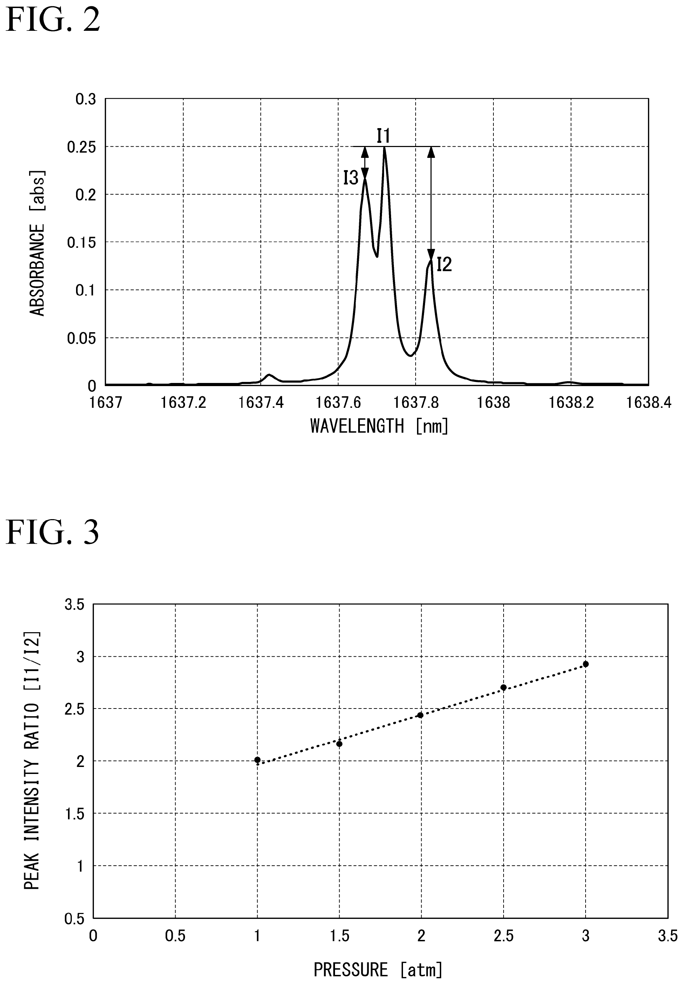

The absorbance data analysis unit 21 performs a spectral analysis of data of the absorbance measured by the absorbance measurement device 10 (absorbance data analysis process). FIG. 2 is a graph showing an example of the absorption spectrum of methane gas according to the embodiment. As shown in FIG. 2, the absorption spectrum of methane gas exhibits a plurality of characteristic peak values I1, I2, and I3 in the wavelength range from infrared ray to near-infrared ray.

The peak detection unit 22 detects two peak values from the absorbance spectrum analyzed by the absorbance data analysis unit 21 (peak detection process). Specifically, the peak detection unit 22 detects two adjacent peak values, including the highest peak value I1, from the absorbance spectrum analyzed by the absorbance data analysis unit 21. By detecting the peak values of two adjacent points, the detection range of the absorbance spectrum can be narrowed, reducing the computational processing load. Further, the peak value can be detected from the point where the slope of the absorbance becomes zero or changes from positive to negative.

As shown in FIG. 2, if multiple pairs of adjacent peak values (for example, the pair of I1 and I2, and the pair of I1 and I3) are detected within a predetermined wavelength range, the peak detection unit 22 selects the pair with the largest difference between the peak values of two adjacent points (the pairs of 11 and 12). Accordingly, the intensity ratio (I1/I2) of the peak value, as described later, becomes more pronounced, and the subsequent pressure estimation accuracy improves. Further, if the specific wavelength at which characteristic peak values appear is known in advance, the set of two peak values can be determined to match the set of specific wavelengths.

The pressure calculation unit 23 calculates the pressure inside the reaction vessel 2 based on the intensity ratio (I1/I2) of the two peak values detected by the peak detection unit 22 (pressure calculation process). FIG. 3 is a graph showing the relationship between the intensity ratio (I1/I2) of the peak values of two points and the pressure according to the embodiment. As shown in FIG. 3, there is a correlation between the intensity ratio (I1/I2) of the two peak values and the pressure. The pressure calculation unit 23 calculates the pressure during spectrum acquisition based on the relationship between the intensity ratio (I1/I2) of the two peak values and the pressure shown in FIG. 3.

The gas concentration calculation unit 24 calculates the gas concentration based on the peak value of the absorbance at a specific wavelength (I2 in the embodiment) analyzed by the absorbance data analysis unit 21 and the pressure inside the reaction vessel 2 calculated by the pressure calculation unit 23 (gas concentration calculation process). Further, the peak value of the absorbance for the specific wavelength mentioned above could be I1, but since there is another peak value, I3, nearby, it may be affected by that. For this reason, the gas concentration calculation unit 24 refers to 12.

The gas concentration calculation unit 24 includes a peak value standardization unit 24a that converts and normalizes the peak value of the absorbance at a specific wavelength (I2 in the embodiment) analyzed by the absorbance data analysis unit 21 to a peak value at a predetermined standard pressure based on the pressure inside the reaction vessel 2 calculated by the pressure calculation unit 23, and a data checking unit 24b that compares the normalized peak value of the absorbance with table data in which the peak value of the absorbance at a standard pressure stored in advance corresponds to the gas concentration.

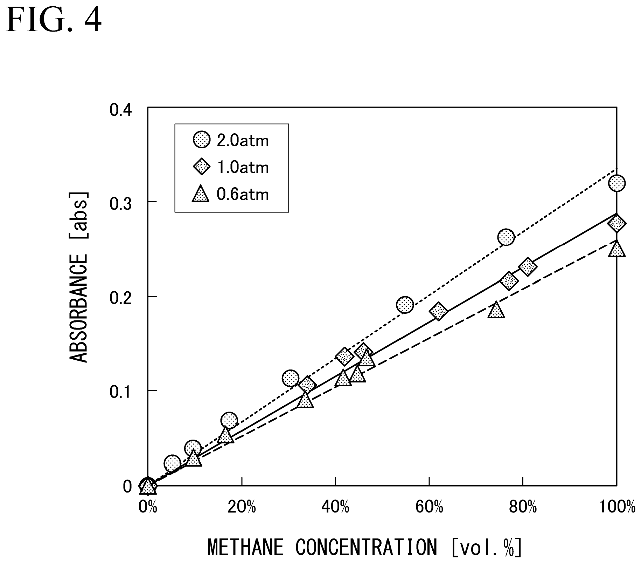

FIG. 4 is a graph showing a relationship between methane concentration and absorbance at each pressure according to the embodiment. As shown in FIG. 4, the methane concentration and the absorbance exhibit linearity with different slopes depending on the pressure. That is, when the methane concentration is constant, the ratio of absorbance to pressure at each pressure is constant. Accordingly, the peak value standardization unit 24a can normalize the absorbance at standard pressure (here assumed to be 1 atm) and convert the absorbance (I2 in this embodiment) to the peak value at 1 atm. Then, the data checking unit 24b stores table data that shows the relationship between a methane concentration at 1 atm and a calibration curve of the absorbance, and calculates the methane concentration from the absorbance converted to 1 atm, which was obtained by the peak value standardization unit 24a.

The concentration output unit 12 outputs the methane gas concentration calculated by the gas concentration calculation unit 24 (gas concentration output process). The concentration output unit 12 is, for example, a display device. Further, the concentration output unit 12 may output not only the concentration of methane gas, but also the pressure inside the reaction vessel 2, which is determined during the calculation process.

In this way, according to the gas measurement device 5 of the embodiment described above, the pressure inside the reaction vessel 2 can be estimated by comparing the intensity ratio of the peak values of two adjacent absorption points in the absorbance spectrum. Accordingly, the pressure inside the reaction vessel 2 can be estimated without using the pressure sensor. In addition, there is no need to separately install a gas cell for a standard gas and a gas cell for enclosing the standard gas, and the pressure inside the reaction vessel 2 can be estimated using only the acquired spectral data. In addition, since the absorbance, which is pressure-dependent, can be corrected using the estimated pressure, it is possible to compare the gas concentrations under the same pressure condition.

As described above, the gas measurement device 5 according to the embodiment includes the absorbance measurement device 10 configured to irradiate detection light into the reaction vessel 2 that generates methane gas and measure the absorbance of the detection light that has passed through the gas atmosphere inside the reaction vessel 2, the absorbance data analysis unit 21 configured to spectrally analyze data of the absorbance measured by the absorbance measurement device 10, the peak detection unit 22 configured to detect two peak values 11 and 12 from the absorbance spectrum analyzed by the absorbance data analysis unit 21, and the pressure calculation unit 23 configured to calculate the pressure inside the reaction vessel 2 based on the intensity ratio (11/12) of the two peak values detected by the peak detection unit 22.

According to this configuration, inside the reaction vessel 2, where the pressure changes, the specific gas can be measured without installing the pressure sensor. That is, since the pressure can be estimated from the peak value of absorbance, there is no need to install the pressure sensor. In addition, the removal of the pressure sensor during heat treatment, such as sterilization inside the reaction vessel 2, becomes unnecessary. In addition, since there is no need to remove the gas sample as in gas chromatography, contamination of the reaction vessel with impurities can be prevented. Further, it is possible to accurately estimate a specific gas concentration inside the reaction vessel where the pressure is changing.

In addition, the gas measurement method according to the embodiment includes the absorption measurement process of irradiating detection light into the reaction vessel 2 that generates gas and measuring the absorbance of the detection light that has passed through the gas atmosphere inside the reaction vessel 2, data of the absorbance analysis process of spectrally analyzing data of the absorbance measured in the absorption measurement process, the peak detection process of detecting two peak values I1 and I2 from the absorbance spectrum analyzed in data of the absorbance analysis process, and the pressure calculation process of calculating the pressure inside the reaction vessel 2 based on the intensity ratio (I1/I2) of the two peak values detected in the peak detection process.

According to this configuration, similarly, inside the reaction vessel 2, where the pressure changes, the specific gas can be measured without installing the pressure sensor.

Hereinabove, while the preferred embodiment of the present invention has been described with reference to the drawings, the present invention is not limited to the above-mentioned embodiment. The various shapes and combinations of the constituent members shown in the above-mentioned embodiment are merely examples, and can be modified in various ways based on design requirements, etc., without departing from the spirit of the present invention.

For example, the measurement target is not limited to methane gas. Similar to the methane gas mentioned above, carbide hydrogen and nitrogen compounds, as well as sulfur compounds, which exhibit near-infrared and infrared absorption, can also be used as measurement targets. Further, if the relationship between the peak intensity ratio (I1/I2) and the pressure, and the relationship between the gas concentration and the absorbance at each pressure are known, pressure correction can be performed without using the pressure sensor, and the concentration of the gas can be quantitatively evaluated. Therefore, the measurement target is not limited to gases that exhibit near-infrared and infrared absorption.

In addition, for example, the peak detection unit 22 is not limited to a configuration that detects only peak values of two points. For example, the peak detection unit 22 may initially detect peak values of three or more points, select the two most optimal points, and use those peak values. Further, the peak detection unit 22 may use values such as 1 or a plurality of peak values that were not selected to perform correction on the pressure or gas concentration.

Further, some or all of the above-mentioned embodiments may also be described as follows, but are not limited to the following supplementary statement.

### (Supplementary Statement 1)

A gas measurement device including:
an absorbance measurement device configured to irradiate detection light into a reaction vessel that generates a gas, and measure absorbance of the detection light that has passed through a gas atmosphere inside the reaction vessel;
an absorbance data analysis unit configured to spectrally analyze data of the absorbance measured by the absorbance measurement device;
a peak detection unit configured to detect a plurality of peak values from the absorbance spectrum analyzed by the absorbance data analysis unit; and
a gas concentration output unit configured to output a concentration of the gas inside the reaction vessel based on an intensity ratio of two peak values of the plurality of peak values detected by the peak detection unit.

### REFERENCE SIGNS LIST

1 Methane generating device
2 Reaction vessel
3 Gas supply device
3a Valve
4 Generated gas extraction device
4a Valve
5 Gas measurement device
6 Culture solution
7 Air-liquid interface
8 Stirring mechanism
10 Absorbance measurement device
11 Information processing unit
12 Concentration output unit
20 Device control unit
21 Absorbance data analysis unit
22 Peak detection unit
23 Pressure calculation unit
24 Gas concentration calculation unit
24a Peak value standardization unit
24b... data checking unit
CO₂ Carbon dioxide
H₂ Hydrogen gas
I1 Peak value
I2 Peak value
I3 Peak value

## Claims

1. A gas measurement device comprising:
an absorbance measurement device configured to radiate detection light into a reaction vessel that generates a gas and measure an absorbance of the detection light that has passed through a gas atmosphere inside the reaction vessel;
an absorbance data analysis unit configured to spectrally analyze data of the absorbance measured by the absorbance measurement device;
a peak detection unit configured to detect a plurality of peak values from the absorbance spectrum analyzed by the absorbance data analysis unit; and
a pressure calculation unit configured to calculate the pressure inside the reaction vessel based on an intensity ratio of the two peak values of the plurality of peak values detected by the peak detection unit.

2. The gas measurement device according to claim 1, comprising a gas concentration calculation unit configured to calculate a concentration of the gas based on the peak value of the absorbance with a specific wavelength analyzed by the absorbance data analysis unit and the pressure inside the reaction vessel calculated by the pressure calculation unit.

3. The gas measurement device according to claim 2, comprising a gas concentration output unit configured to output the concentration of the gas calculated by the gas concentration calculation unit.

4. The gas measurement device according to claim 2, wherein the gas concentration calculation unit comprises a data checking unit configured to compare table data, which shows the correspondence between the peak value of the absorbance at a predetermined standard pressure stored in advance and the gas concentration, with the peak value of the absorbance.

5. The gas measurement device according to claim 4, wherein the gas concentration calculation unit includes a peak value standardization unit configured to convert the peak value of the absorbance at a specific wavelength analyzed by the absorbance data analysis unit into a peak value at a predetermined standard pressure from the pressure inside the reaction vessel calculated by the pressure calculation unit, and normalize the peak value, and
the data checking unit compares the peak value of the standardized absorbance with the table data, which corresponds to the gas concentration and the peak value of the absorbance at the standard pressure stored in advance.

6. The gas measurement device according to any one of claims 1 to 5, wherein the peak detection unit detects the peak values of two adjacent points from the absorbance spectrum analyzed by the absorbance data analysis unit.

7. The gas measurement device according to claim 6, wherein, when the peak detection unit detects multiple pairs of adjacent peak values within a predetermined wavelength range, the peak detection unit selects the pair with the largest difference between the peak values of two adjacent points.

8. A gas measurement method comprising:
an absorbance measurement process of radiating detection light into a reaction vessel that generates a gas and measuring an absorbance of the detection light that has passed through a gas atmosphere inside the reaction vessel;
an absorbance data analysis process of spectrally analyzing data of the absorbance measured in the absorbance measurement process;
a peak detection process of detecting a plurality of peak values from the absorbance spectrum analyzed in the absorbance data analysis process; and
a pressure calculation process of calculating the pressure inside the reaction vessel based on an intensity ratio of the two peak values in the plurality of peak values detected in the peak detection process.
